**Europäisches Patentamt**

**European Patent Office**

**Office européen des brevets**

(11) Veröffentlichungsnummer : **0 379 929 B1**

# (12) EUROPÄISCHE PATENTSCHRIFT

(45) Veröffentlichungstag der Patentschrift :
23.12.92 Patentblatt 92/52

(51) Int. Cl.$^5$ : **A61F 5/04**

(21) Anmeldenummer : **90100790.6**

(22) Anmeldetag : **16.01.90**

(54) **Clavicula-Bandage.**

(30) Priorität : **24.01.89 DE 3901918**

(43) Veröffentlichungstag der Anmeldung :
**01.08.90 Patentblatt 90/31**

(45) Bekanntmachung des Hinweises auf die
Patenterteilung :
**23.12.92 Patentblatt 92/52**

(84) Benannte Vertragsstaaten :
**AT BE CH DE ES FR GB IT LI NL SE**

(56) Entgegenhaltungen :
**BE-A- 746 928**
**DE-C- 259 349**
**DE-U- 8 900 721**
**US-A- 3 856 004**
**US-A- 3 897 776**

(73) Patentinhaber : **Beiersdorf Aktiengesellschaft**
**Unnastrasse 48**
**W-2000 Hamburg 20 (DE)**

(72) Erfinder : **Koopmann, Jens-Wolfgang**
**Gerhart-Hauptmannstrasse 7**
**W-2121 Reppenstedt b/Lüneburg (DE)**

**Beschreibung**

Die Erfindung betrifft eine Bandage zur Behandlung von Verletzungen des Schlüsselbeins (Clavicula), d.h. in erster Linie von Frakturen, die meist als Schräg- oder Splitterbrüche im mittleren Drittel der Clavicula an der Stelle ihrer stärksten Biegung entstehen. Ziel ist es dabei vor allem, die Clavicula in Längsrichtung zu dehnen und ruhig zu stellen, damit die Bruchenden nicht aneinanderreiben sondern passend zusammenwachsen können.

Für die Bandagierung derartiger Verletzungen wird vielfach ein sog. "Rucksack"-Verband angelegt, der üblicherweise aus einem z.T. mit Polsterwatte gefüllten Trikotschlauch oder speziellen Schlauchbandagen vom Klinikpersonal selbst hergestellt wird. Diese Verbände sind zwar recht preisgünstig, haben jedoch u.a. die Nachteile, daß das Material sich unter Zug ausdehnt, wobei die Position der Knochenteile sich wieder verschieben kann, pro Heilungsverlauf mehrere Verbände zeitaufwendig hergestellt und die Verbände auf dem Rücken zweifach geknotet werden müssen, was in Rückenlage des Patienten unangenehme Druckstellen hervorruft.

Außerdem gibt es schon eine Reihe von Fertigbandagen, die jedoch noch nicht alle an eine solche Bandage gestellten Anforderungen zur vollen Zufriedenheit erfüllen. Sie sind z.T. sehr aufwendig und auftragend, haben komplizierte Verschlußsysteme und Metallspangen, die zu Allergien führen können, und lassen sich gar nicht oder nur schwierig nachspannen.

Aus der US-A-3 856 004 ist eine Clavicula Bandage bekannt, die aus einem Streifen besteht, der in Längsrichtung bis auf ein Reststück in zwei Bänder aufgeteilt ist. Auf dem Reststück ist ein gleichseitiges Dreieck befestigt, durch das die Enden der Bänder gezogen und auf sich selbst befestigt werden. Eine derartige Bandage hat jedoch, aufgrund ihres Aufbaus und weil ihre beiden Bänder nicht unabhängig voneinander sind, den Nachteil, daß beim Spannen der Bänder das Dreieck automatisch nach unten gezogen wird und gleichzeitig die auf der Schulter aufliegenden Bänder nach innen, d.h. zum Nacken hin, gezogen werden. Dies führt zu keinem optimalen Heilungsverlauf, da eine gute Reposition der Fraktur es erfordert, daß die Bänder nicht auf die Bruchstelle drücken sondern auf dem äußeren Drittel des Schlüsselbeins zu liegen kommen und in dieser Position verbleiben.

Aufgabe der Erfindung war es deshalb, eine Clavicula-Bandage zu entwickeln, die bei hoher Therapiesicherheit, d.h. anatomisch guter Sitz ohne Verrutschen und Lockern, einfach anzulegen und für den Patienten angenehm zu tragen ist sowie, falls notwendig, im Heilungsverlauf leicht nachgestellt werden kann.

Gelöst wird diese Aufgabe durch eine Clavicula-Bandage mit zwei in ihrer Länge einstellbaren Bändern, die an ihren freien Enden Mittel zur Schlaufenbildung aufweisen, und dadurch gekennzeichnet ist, daß die Bänder jeweils mit ihrem anderen Ende an einem Ring unabhängig voneinander befestigt sind - und damit über diesen Ring miteinander verbunden sind - und diese Bänder aus einem im wesentlichen nicht dehnbaren Material bestehen.

Durch eine derartige Anordnung der Bänder wird erreicht, daß diese über den Ring winkelverschieblich bzw. winkeleinstellbar zueinander verbunden sind.

In einer besonders bevorzugten Ausführungsform der Erfindung bestehen diese Mittel zur Schlaufenbildung aus einem Klettverschlußsystem. In der Praxis ist dabei das Verschlußsystem vorzugsweise in der Weise ausgebildet, daß sich an den freien Enden der Bänder auf ihrer körperabgewandten Außenfläche ein Klettenabschnitt befindet, der das eine Verschlußteil des Klettverschlusses bildet und aus im Abstand voneinander angeordneten, aufwärts gerichteten Häkchen besteht. Ebenfalls auf der körperabgewandten Seite der Bänder befindet sich im Anschluß an diesen Klettenabschnitt zumindest auf einem Teilabschnitt ihrer Länge klettbares Material, welches als das andere Teil des Klettverschlusses fungiert, indem die Häkchen auf ihm fest zu haften vermögen.

Es hat sich als vorteilhaft und nützlich erwiesen, daß dieser klettbare Teilabschnitt mindestens 1/6 der Länge der Bänder ausmacht. Besonders einfach gestaltet sich die Herstellung der Bandage, wenn die Bänder auf ihrer körperabgewandten Außenfläche über die ganze Länge aus einem Material bestehen, auf welchem die Häkchen des anderen Verschlußteils haften.

Der Ring in der Mitte der Bandage, an dem die Bänder befestigt sind - z.B. mittels einer kleinen Schlaufe, deren Ende festgenäht ist - und durch welchen ihre freien Enden beim Anlegen der Bandage hindurchgeführt werden, ist vorzugsweise als flacher, runder Ring aus Plastikmaterial ausgebildet. Er kann jedoch auch ein Vieleck, z.B. Sechs- oder Achteck, darstellen.

Auf der dem Körper zugewandten Seite der Bänder befindet sich etwa in ihrem Mittelabschnitt eine gepolsterte Zone. Da dieser Abschnitt im angelegten Zustand der Bandage im Unterachselbereich zum Anliegen kommt, sollte die Polsterung aus einem gut saugfähigen Material, vorzugsweise aus Baumwolle, bestehen.

Die ganze Bandage besteht im übrigen in einer vorzugsweisen Ausführungsform aus einem Schlingengengewebe oder aufgerauhten Gewebe aus Polyamid, z.B. gekräuseltem Nylon, in Doppellage mit einer Zwischenlage aus Schaumstoff, die durch mehrere längsgeführte Steppnähte fixiert ist. Das Material ist zugfest und praktisch nicht dehnbar, angenehm auf der Haut und leicht waschbar.

Als Hilfsmittel für den behandelnden Arzt kann außerdem auf der Bandage, vorzugsweise auf ihrer Innenseite, eine Graduierung angebracht sein, die es ermöglicht, die Länge und damit die Spannung der Bänder anzugeben, zu überprüfen und auch zu reproduzieren, wenn die Bandage zwischenzeitlich einmal abgenommen werden mußte.

Anhand der Abbildungen soll die erfindungsgemäße Bandage beispielhaft näher erläutert werden.

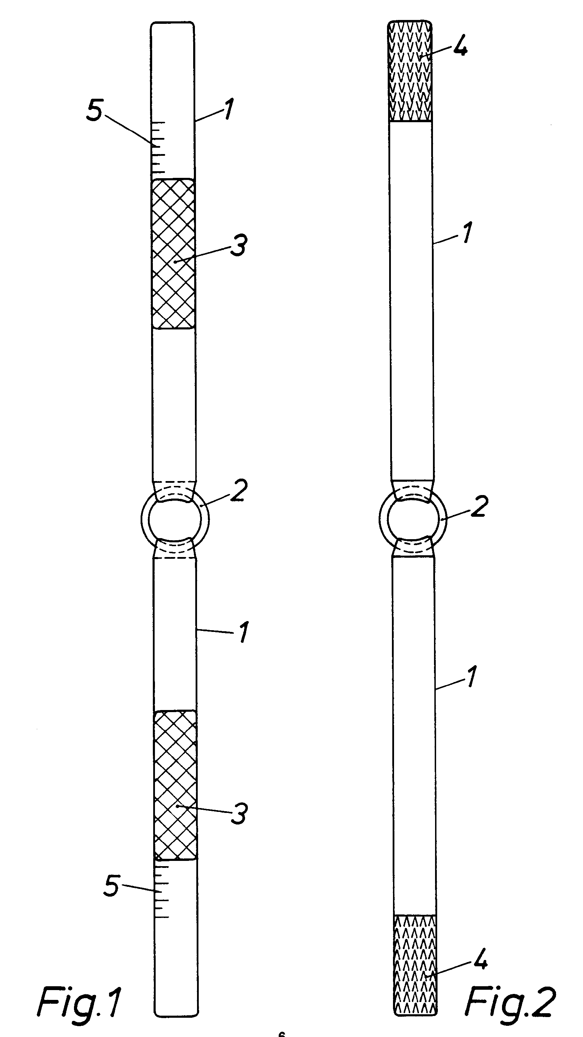

Figur 1 zeigt schematisch die Bandage von der körperzugewandten Seite aus, mit den beiden Bändern (1), dem Verbindungsring (2), den Polsterungen (3) und einer Graduierung (5).

Figur 2 zeigt die gleiche Bandage von der körperabgewandten Seite mit den Bändern (1), dem Verbindungsring (2) und den Klettenabschnitten mit den Häkchen (4). Die Außenfläche der Bandage soll hier über die ganze Länge aus klettbarem Material bestehen.

Die Bandage hat in den verschiedenen Größen eine Gesamtlänge von etwa 1 m bis 1.80 m, wobei die Klettenabschnitte etwa 10 bis 15 cm und die Polsterabschnitte etwa 20 bis 40 cm lang sind, eine Bandbreite von etwa 5 cm und eine Banddicke von etwa 1 cm. Der Ring hat einen Außendurchmesser von etwa 8 cm und einen Innendurchmesser von etwa 7 cm.

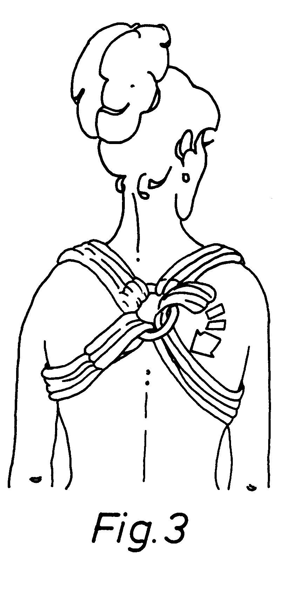

Figur 3 zeigt, wie die Bandage angelegt wird, wobei der Vorgang beinahe abgeschlossen ist. Das Anlegen geschieht im wesentlichen in der Weise, daß die Bandage mit dem Ring im Rücken um den Nacken des Patienten gelegt wird. Dann werden die beiden freien Enden der Bänder unter den Achseln durch von vorne nach hinten gelegt und nacheinander, d.h. die gesunde Seite zuerst, durch den Ring von innen nach außen hindurchgezogen und mit den Klettverschlüssen unter Schlaufenbildung auf sich selbst befestigt. Anschließend werden in der Regel beide Klettverschlüsse noch einmal kurz gelöst zum gleichmäßigen Spannen und wenn die optimale Spannung und der richtige Sitz der Bandage erreicht sind, die Klettverschlüsse endgültig geschlossen.

Die besonderen Vorteile der erfindungsgemäßen Clavicula-Bandage sind insbesondere ihre hohe Therapiesicherheit, da das zugfeste und nicht dehnbare Bandmaterial, das kaum ein Nachspannen erforderlich macht, für eine gute Stabilität und perfekten Sitz sorgt, sowie ihr einfaches Anlegen durch die Klettverschlüsse. Hierbei ist vor allem praktisch, daß die Außenseite der Bänder über einen größeren Teilabschnitt ihrer Länge klettbar ist, so daß wegen dieser variablen Verstellbarkeit der Bänderlänge nur wenige Bandagengrößen für die verschiedensten Patienten notwendig sind. Einen weiteren Vorteil stellt der flache Verbindungsring dar, der nicht aufträgt und den Patienten beim Liegen nicht stört sowie außerdem - was sehr wesentlich ist - bewirkt, daß die Bänder jeweils auf das äußere Drittel des Schlüsselbeins zu liegen kommen und damit nicht direkt auf die Bruchstelle drücken, die in der Regel etwa in seiner Mitte liegt.

Dies führt zu einer besseren Reposition der Fraktur als bei den herkömmlichen Bandagen.

**Patentansprüche**

1. Clavicula-Bandage mit zwei in ihrer Länge einstellbaren Bändern (1), die an ihren freien Enden Mittel zur Schlaufenbildung aufweisen, dadurch gekennzeichnet, daß die Bänder jeweils mit ihrem anderen Ende an einem Ring (2) unabhängig voneinander befestigt sind und damit über diesen Ring (2) miteinander verbunden sind und aus im wesentlichen nicht dehnbarem Material bestehen.

2. Bandage gemäß Anspruch 1, dadurch gekennzeichnet, daß die Mittel zur Schlaufenbildung aus einem Klettverschlußsystem bestehen.

3. Bandage gemäß Anspruch 2, dadurch gekennzeichnet, daß das Klettverschlußsystem in der Weise ausgebildet ist, daß sich am freien Ende der Bänder auf ihrer körperabgewandten Außenfläche ein Klettenabschnitt befindet, der das eine Verschlußteil des Klettverschlusses bildet und aus im Abstand voneinander angeordneten aufwärts gerichteten Häkchen besteht, und daß sich im Anschluß daran zumindest auf einem Teilabschnitt der Außenfläche klettbares Material befindet, welches als das andere Teil des Klettverschlusses fungiert, indem die Häkchen auf ihm zu haften vermögen.

4. Bandage gemäß Anspruch 3, dadurch gekennzeichnet, daß der klettbare Teilabschnitt mindestens ein Sechstel der Länge der Bänder ausmacht.

5. Bandage gemäß Anspruch 3, dadurch gekennzeichnet, daß die Bänder auf ihrer körperabgewandten Außenfläche über die ganze Länge aus einem Material bestehen, auf welchem die Häkchen des anderen Verschlußteils haften.

6. Bandage gemäß einem oder mehreren der Ansprüche 1 bis 5, dadurch gekennzeichnet, daß der Ring, an dem die beiden Bänder befestigt sind, als flacher, runder Ring aus Plastikmaterial ausgebildet ist.

7. Bandage gemäß einem oder mehreren der Ansprüche 1 bis 6, dadurch gekennzeichnet, daß die Bänder etwa im Mittelabschnitt ihrer körperzugewandten Innenseite eine Polsterung aus einem schweißaufsaugenden Material, vorzugsweise aus Baumwolle, aufweisen.

8. Bandage gemäß einem oder mehreren der Ansprüche 1 bis 7, dadurch gekennzeichnet, daß auf ihrer Innenseite eine Graduierung angebracht ist.

**Claims**

1. Clavicle bandage, having two straps (1) which are adjustable in their length and have means for forming loops at their free ends, characterised in that the straps are each attached by their other end to a ring (2) independently of one another and are thus connected to one another via said ring (2) and consist essentially of non-stretchable material.

2. Bandage according to Claim 1, characterised in that the means for forming loops consist of a touch-and-close fastener system.

3. Bandage according to Claim 2, characterised in that the touch-and-close fastener system is constructed in such a way that there is a touch-and-close fastener portion at the free end of the straps on their outer surface facing away from the body, which portion forms one fastener part of the touch-and-close fastener and consists of small hooks arranged spaced apart and directed upwards, and in that, adjoining said portion, there is a grippable material at least on a partial portion of the outer surface, which grippable material functions as the other part of the touch-and-close fastener, in that the small hooks are able to adhere to it.

4. Bandage according to Claim 3, characterised in that the grippable partial portion takes up at least one sixth of the length of the straps.

5. Bandage according to Claim 3, characterised in that, on their outer surface facing away from the body, the straps consist over the entire length of a material to which the small hooks of the other fastener part adhere.

6. Bandage according to one or more of Claims 1 to 5, characterised in that the ring, to which the two straps are attached, is constructed as a flat, round ring made of plastic material.

7. Bandage according to one or more of Claims 1 to 6, characterised in that, approximately in the central portion of their inside facing the body, the straps have a padding made of a perspiration-absorbent material, preferably made of cotton.

8. Bandage according to one or more of Claims 1 to 7, characterised in that a graduation is provided on its inside.

**Revendications**

1. Bandage claviculaire comprenant deux bandelettes (1) ajustables dans leur longueur, qui présentent à leurs extrémités libres des moyens pour former une boucle, caractérisé en ce que les bandelettes sont chacune fixées indépendamment l'une de l'autre par leur autre extrémité à un anneau (2) et sont donc liées entre elles par l'intermédiaire de cet anneau (2) et en ce que ces bandelettes consistent en une matière essentiellement indéformable.

2. Bandage suivant la revendication 1, caractérisé en ce que les moyens pour former une boucle consistent en un système d'attache à crochets.

3. Bandage selon la revendication 2, caractérisé en ce que le système d'attache à crochets est constitué de telle manière, qu'aux extrémités libres des bandelettes et sur leur face externe éloignée du corps, se trouve une section à crochets qui forme l'une des pièces d'attache de l'attache à crochets, et consiste en des petits crochets pointés vers le haut, disposés à une certaine distance les uns des autres, et en ce que, adjoignant cette section à crochets, se trouve, au moins sur une section de la face externe, une matière accrochable qui fait fonction de deuxième pièce de l'attache à crochets, en ce que les crochets peuvent s'agripper fermement à elle.

4. Bandage suivant la revendication 3, caractérisé en ce que la section accrochable mesure au moins un sixième de la longueur des bandelettes.

5. Bandage suivant la revendication 3, caractérisé en ce que les bandelettes consistent sur toute la longueur de leur face externe éloignée du corps, en une matière à laquelle les petits crocheta de l'autre pièce d'attache peuvent s'agripper.

6. Bandage suivant une ou plusieurs des revendications 1 à 5, caractérisé en ce que l'anneau auquel sont fixées les deux bandelettes, est un anneau plat et circulaire en matière plastique.

7. Bandage suivant une ou plusieurs des revendications 1 à 6, caractérisé en ce que les bandelettes présentent, environ dans la section centrale de leur côté intérieur tourné vers le corps, un rembourrage en matière absorbant la sueur, de préférence du coton.

8. Bandage suivant une ou plusieurs des revendications 1 à 7, caractérisé en ce qu'une graduation est ajoutée sur son côté intérieur.

Fig.1

Fig.2

Fig.3